(19) Europäisches Patentamt
European Patent Office
Office européen des brevets

(11) Numéro de publication: **0 286 522 B1**

(12) **FASCICULE DE BREVET EUROPEEN**

(45) Date de publication de fascicule du brevet: **11.11.92**

(51) Int. Cl.5: **C07D 493/04**, //(C07D493/04, 319:00,319:00)

(21) Numéro de dépôt: **88400794.9**

(22) Date de dépôt: **01.04.88**

# (54) Diacétals d'alditols, de grande pureté et exempts de traces de solvants organiques, et procédés pour leur préparation.

(30) Priorité: **07.04.87 FR 8704884**

(43) Date de publication de la demande:
**12.10.88 Bulletin 88/41**

(45) Mention de la délivrance du brevet:
**11.11.92 Bulletin 92/46**

(84) Etats contractants désignés:
**AT BE DE ES FR GB IT NL**

(56) Documents cités:
**FR-A- 2 356 619**
**FR-A- 2 486 080**

**CHEMICAL ABSTRACTS, vol. 97, no. 15, 11 octobre 1982, page 763, résumé no. 128001w, Columbus, Ohio, US; & JP - A - 82 18 682**

**CHEMICAL ABSTRACTS, vol. 104, no. 17, 28 avril 1986, page 745, résumé no. 149347s, Columbus, Ohio, US; & JP - A - 60 199 891**

**CHEMICAL ABSTRACTS, vol. 89, no. 19, 6 novembre 1978, pages 526-527, résumé no. 163005g, Columbus, Ohio, US; A.I. BLAGA et al.: "Acid-catalyzed acetalization. I. The influence of kind and concentration of acid catalysts on synthesis of bis-1,1-(2',2'-ethylhexyloxy)-2-methylpropane"**

(73) Titulaire: **Roquette Frères**

**F-62136 Lestrem(FR)**

(72) Inventeur: **Salome, Jean-Paul**
**61 Route d'Estaires**
**F-59232 Vieux Berquin(FR)**
Inventeur: **Fleche Guy**
**49 Rue Georges Charlet Le Sart**
**F.59660 Merville(FR)**

(74) Mandataire: **Koch, Gustave et al**
**Cabinet PLASSERAUD 84, rue d'Amsterdam**
**F-75009 Paris(FR)**

**EP 0 286 522 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention a pour objet des diacétals résultant de la déshydrocondensation d'un alditol à 5 ou 6 atomes de carbone et d'un aldéhyde benzoïque, ces diacétals présentant une pureté d'au moins 95% et étant exempts de traces de solvants organiques.

Par le document antérieur CHEMICAL ABSTRACTS, Vol. 104, No.17, 28-4-1986, résumé no. 149347s, on connaît déjà les dérivés éthylé et méthylé du dibenzylidène-sorbitol.

Les diacétals conformes à la présente invention sont ceux résultant de la déshydrocondensation d'un alditol à 5 ou 6 atomes de carbone et d'un aldéhyde benzoïque, en particulier le benzaldéhyde, éventuellement substitué par au moins un groupe alcoyle de 1 à 4 atomes de carbone ou par un halogène, à l'exclusion du bis(para-éthylbenzylidène)sorbitol ou BEBS et du bis(para-méthylbenzylidène)-sorbitol ou BMBS, ces diacétals qui présentent une pureté d'au moins 95% et qui sont exempts de traces de solvants organiques, étant susceptibles d'être préparés par mise en oeuvre du procédé selon lequel l'acétalisation est réalisée par mélange des réactifs sous agitation, le mélange réactionnel étant ensuite neutralisé par une base et la phase solide séparée de la phase liquide et lavée à l'eau chaude, le rapport molaire initial de l'aldéhyde benzoïque à l'alditol étant inférieur à 2/1 et la température de réaction inférieure à 45 °C environ, ledit procédé étant caractérisé par le fait que:

- le catalyseur acide est un acide arylsulfonique et
- le rapport molaire initial de l'acide arylsulfonique à l'aldéhyde benzoïque est supérieur à 0,6.

Le dibenzylidène-sorbitol ou DBS est préparé selon l'invention par déshydrocondensation du sorbitol et du benzaldéhyde en présence d'un catalyseur acide.

L'action d'un aldéhyde benzoïque sur un alditol à 5 ou 6 atomes de carbone est une réaction d'acétalisation catalysée par les protons et conduisant, par des déshydrocondensations successives, à la formation du monoacétal, diacétal, et, le cas échéant, triacétal correspondants.

Par exemple, dans le cas de l'action du benzaldéhyde sur le sorbitol, on obtient successivement la formation des dérivés suivants :

- 1,3-monobenzalsorbitol ou monobenzylidène-sorbitol (MBS),
- 1,3-2,4-dibenzalsorbitol ou dibenzylidène-sorbitol (DBS) et
- 1,3-2,4-5,6-tribenzalsorbitol ou tribenzylidènesorbitol (TBS).

De manière analogue, le dibenzylidène-xylitol ou DBX est préparé selon l'invention par déshydrocondensation du xylitol et du benzaldéhyde en présence d'un catalyseur acide.

Dans tous les cas, le produit de grande pureté concerné par l'invention est le composé disubstitué, en particulier le DBS et le DBX. Les composés mono- et trisubstitués tels que respectivement le MBS et le TBS, bien que pouvant être utilisés dans certaines applications, sont considérés ici comme des "impuretés".

Les composés disubstitués selon l'invention peuvent être utilisés notamment en tant qu'agents de gélification des liquides organiques ou agents d'épaississement.

En ce qui concerne plus particulièrement le DBS et le DBX notamment, ils peuvent être utilisés en vue de conférer à certains polymères des propriétés remarquables (transparence, résistance aux chocs, etc.), c'est-à-dire notamment en tant qu'agents de clarification et/ou de stabilisation des polyoléfines.

Dans cette optique, le DBS est communément incorporé dans des compositions à base de polypropylène ou de polyéthylène destinées à la fabrication d'articles transparents (feuilles, emballages, récipients) à usage alimentaire, médical ou autres.

Pour des raisons d'ordre technique et/ou législatif il est généralement requis, pour les applications du DBS susmentionnées, une pureté en DBS d'au moins 95%.

Il est rappelé à ce propos que la pureté en question est définie comme suit :

$$\text{pureté du DBS} = \frac{\text{masse de DBS}}{\text{masse de DBS} + \text{masse de TBS}} \times 100,$$

les masses de DBS et de TBS étant calculées sur l'extrait sec obtenu après séparation des phases liquide et solide du milieu réactionnel et lavage à l'eau chaude de la phase solide obtenue, lavage qui élimine notamment le sorbitol résiduel et le MBS éventuellement présents, sans avoir d'effet sur le DBS et le TBS qui sont insolubles dans l'eau chaude.

Quels que soient les procédés de préparation du DBS décrits dans l'art antérieur, aucun ne permet de façon simple, économique et reproductible d'obtenir un DBS brut présentant une pureté d'au moins 95 %. Il

existe donc le besoin de disposer d'un procédé simple et facile à mettre en oeuvre, permettant d'obtenir du DBS brut présentant une telle pureté.

Classiquement, on distingue deux types de méthodes de préparation du DBS selon la nature du milieu réactionnel : la méthode utilisant au moins un solvant organique et appelée "méthode solvant" et la méthode utilisant l'eau comme solvant unique et appelée "méthode aqueuse".

La méthode solvant telle que décrite, par exemple, dans le brevet FR 2 065 001 semble permettre d'obtenir un produit dans lequel le DBS serait prédominant en jouant sur le rapport molaire initial benzaldéhyde/sorbitol, et il est indiqué que "l'utilisation de deux moles de benzaldéhyde par mole de sorbitol a pour résultat un produit qui ne se compose pratiquement que de dibenzylidène-sorbitol". Un tel résultat apparaît satisfaisant. Toutefois aucune donnée précise concernant la pureté n'est fournie et le mode opératoire utilisé est complexe.

En effet, le brevet FR 2 065 001 préconise l'utilisation du cyclohexane en tant que milieu réactionnel et l'utilisation d'un catalyseur acide minéral.

Selon ce document l'eau aurait une influence nuisible sur la vitesse et le rendement de la réaction ; c'est pourquoi elle est éliminée par distillation d'un mélange azéotropique cyclohexane/eau, avec recyclage du cyclohexane en continu.

La distillation du mélange azéotropique cyclohexane/eau et le recyclage du cyclohexane constituent des étapes de mise en oeuvre compliquées.

Le brevet EP 51 681 du même demandeur décrit un procédé qui devrait donner des performances améliorées en mettant en oeuvre un système d'agitation forcée permettant, dans des conditions assez voisines de celles décrites dans le brevet français précité (et notamment une température réactionnelle de l'ordre de 70-80 ° C), d'obtenir un DBS brut d'une pureté supérieure à 90 % et pouvant atteindre 99 % dans des cas particuliers.

Ce brevet étend l'usage des solvants aux hydrocarbures saturés et revendique l'emploi impératif d'un solvant polaire organique, soluble dans l'eau, sans lequel la réaction n'a pratiquement pas lieu. Il mentionne l'utilisation indifférente de catalyseurs acides minéraux ou organiques, les résultats (pureté et rendement) les plus remarquables étant obtenus en présence d'acide sulfurique.

Le système solvant, basé sur l'élimination azéotropique de l'eau, présente cependant l'inconvénient d'être assez complexe et ce d'autant plus que le solvant polaire, lequel est indispensable et est souvent présent en une forte proportion, doit également être récupéré.

Le coût élevé résultant de l'infrastructure spécifique nécessaire à la mise en oeuvre d'un tel procédé ainsi que le danger d'explosion par exemple lorsqu'une forte proportion de solvant est présente, font qu'un tel système présente, à l'évidence, un certain nombre de désavantages.

En résumé, la "méthode solvant" permet, au moins théoriquement, d'obtenir des puretés en DBS plutôt satisfaisantes, mais nécessite une mise en oeuvre complexe et lourde.

La "méthode aqueuse" développée jusqu'à présent d'une mise en oeuvre relativement plus simple mais ne conduit pas à une pureté suffisante pour la plupart des applications, à savoir une pureté au moins égale à 95 % et si possible de 100 %.

Ainsi, le brevet FR 2 442 850 décrit un procédé de préparation du DBS en milieu aqueux caractérisé par une addition en deux étapes du catalyseur, celui-ci pouvant être un acide minéral ou organique, avec dilution du milieu réactionnel avant la deuxième étape.

La première étape qui utilise une solution de sorbitol très concentrée doit obligatoirement être réalisée à une température de 50 à 70 ° C (comprise de préférence entre 60 et 65 ° C) tandis que la seconde étape impose l'abaissement de la température vers 15-25 ° C. Le passage d'une étape à l'autre apparaît comme délicat et devant avoir lieu à un stade bien particulier de la réaction.

Le DBS brut obtenu présente une pureté maximale qui reste de l'ordre de 90 %.

Il est précisé que l'"on utilise deux moles de benzaldéhyde par mole de sorbitol", mais que cette proportion peut généralement être comprise entre 1,6 et 2,3 moles (de benzaldéhyde par mole de sorbitol).

La demande de brevet FR 2 486 080 décrit deux modes opératoires pour la fabrication du DBS en phase aqueuse en utilisant un acide minéral à titre de catalyseur. Ces deux modes opératoires sont mis en oeuvre à la température ambiante (environ 25 ° C).

Selon le premier mode opératoire A qui n'est pas revendiqué, tous les réactifs sont introduits simultanément. La meilleure pureté obtenue selon ce mode opératoire correspond à un rapport DBS/TBS de 86/14, soit une pureté de 86 % en DBS (exemple 13).

Selon le deuxième mode opératoire B qui est revendiqué, on arrive à augmenter la pureté en ajoutant le benzaldéhyde au milieu réactionnel aqueux de façon très progressive (par exemple en 4 heures).

Selon cette demande de brevet, il est constaté que la pureté en DBS augmente lorsque le rapport du nombre de moles de benzaldéhyde au nombre de moles de sorbitol diminue par rapport à la valeur

stoechiométrique de 2/1.

Il est revendiqué un rapport molaire du D-sorbitol à l'aldéhyde compris entre environ 1/0,75 et environ 1/1,75, soit un rapport molaire de l'aldéhyde au D-sorbitol compris entre environ 0,75 et 1,75.

Toutefois avec ce mode opératoire relativement complexe selon lequel l'aldéhyde est ajouté très progressivement au mélange réactionnel aqueux contenant le sorbitol et l'acide minéral, on ne peut éviter la formation de TBS, et la pureté maximale en DBS réputée atteinte est de 94 %. Dans ce document il est constaté que la pureté en DBS obtenue n'est pas suffisante pour certaines applications industrielles et il est par conséquent proposé d'éliminer le TBS, en partie ou en totalité, par traitement au moyen d'un solvant organique non polaire tel que le trichloro-1,1,1-éthane, ce qui conduirait inévitablement à la présence de traces de ce solvant dans le produit fini.

Il n'existait donc jusqu'à présent aucun procédé permettant d'obtenir, en milieu aqueux, un diacétal d'alditol, et notamment du DBS, ayant une pureté répondant à la plupart des exigences, notamment réglementaires, c'est-à-dire au moins égale à 95 %, sans étape supplémentaire de purification par un solvant organique apportant des traces d'un tel solvant dans le produit fini.

En résumé, selon l'état de la technique il n'était pas possible, même en mettant en oeuvre des procédés complexes et coûteux, d'obtenir des diacétals d'alditols, notamment du DBS, ayant une pureté d'au moins 95 % et dépourvus de traces de solvants organiques. En effet, les documents de l'art antérieur dont certains suggèrent la possibilité d'obtenir un DBS très pur, préconisent obligatoirement l'utilisation, au cours de la préparation ou de la purification pour obtenir la pureté désirée, d'au moins un solvant organique qui laisse obligatoirement des traces dans le produit fini. Ainsi donc, aucun de ces documents ne décrit les diacétals selon l'invention ni même ne donne des indications susceptibles de permettre leur obtention.

Les recherches intensives effectuées par la demanderesse l'ont conduite à mettre au point un procédé en milieu aqueux qui permet d'obtenir un diacétal d'alditol présentant une telle pureté, et qui plus est, sans qu'il soit nécessaire de procéder en plusieurs étapes ou d'ajouter progressivement l'un des réactifs, par exemple l'aldéhyde.

La Société Demanderesse a trouvé qu'il était possible, grâce à une combinaison surprenante et inattendue de paramètres, d'obtenir un diacétal d'alditol, et notamment du DBS et du DBX, en milieu aqueux avec des résultats comparables, voire supérieurs, à ceux communément annoncés pour une "méthode solvant".

Les paramètres concernés par l'invention sont:

- le choix de l'acide utilisé comme catalyseur; il doit s'agir d'un acide arylsulfonique substitué ou non, les acides minéraux présentés comme obligatoires dans le brevet FR 2 486 080 étant exclus comme inappropriés;
- le rapport molaire existant entre l'acide et l'aldéhyde benzoïque.

Plus précisément, l'invention a pour objet un procédé pour la préparation d'un diacétal à partir d'un alditol comprenant 5 ou 6 atomes de carbone et d'un aldéhyde benzoïque en milieu aqueux, en présence d'un catalyseur acide, selon lequel l'acétalisation est réalisée par mélange des réactifs sous agitation, le mélange réactionnel est ensuite neutralisé par une base, la phase solide est séparée de la phase liquide et lavée à l'eau chaude, le rapport molaire initial de l'aldéhyde benzoïque à l'alditol est inférieur à 2/1 et la température de réaction est inférieure à 45 °C environ, lequel procédé est caractérisé par le fait que:

- le catalyseur acide est un acide arylsulfonique et
- le rapport molaire initial de l'acide arylsulfonique à l'aldéhyde benzoïque est supérieur à 0,6.

Il est rappelé que l'on entend par alditol un polyol de formule $HO\text{-}CH_2\text{-}(CHOH)_n\text{-}CH_2OH$. Les alditols utilisés selon l'invention sont ceux dans lesquels n représente 3 ou 4. Il s'agit notamment du sorbitol (n = 4), du mannitol (n = 4) et du xylitol (n = 3).

Selon l'invention, en tant qu'aldéhyde benzoïque on peut utiliser le benzaldéhyde

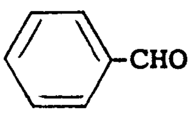

ou des dérivés de substitution de celui-ci, notamment des dérivés dans lesquels le noyau phényle porte au moins un substituant choisi parmi les halogènes et notamment le chlore et les groupes alcoyle inférieur avec 1 à 4 atomes de carbone, de préférence le groupe méthyle ou le groupe éthyle.

L'acide arylsulfonique peut être choisi dans une gamme relativement étendue d'acides dans lesquels le groupe aryle est le groupe phényle, substitué ou non, ou un groupe aryle plus complexe tel que notamment

le groupe naphtyle et on peut alors mettre en oeuvre l'acide naphtalènesulfonique.

En tant qu'acides préférés utilisables selon l'invention, on peut ainsi mentionner :

- l'acide paratoluènesulfonique (désigné ci-après APTS),
- l'acide benzènesulfonique,
- l'acide 5-sulfosalicylique, et
- l'acide naphtalènesulfonique.

L'acide arylsulfonique est de préférence apporté tel quel dans le système réactionnel mais peut également y être introduit sous forme de sel, de préférence de sel de sodium, en présence d'un acide minéral tel que l'acide chlorhydrique, en une quantité suffisante pour libérer l'acide arylsulfonique nécessaire, in situ.

Selon un mode préféré de réalisation, l'invention a pour objet un procédé pour la préparation du dibenzylidène-sorbitol par acétalisation du sorbitol par le benzaldéhyde en milieu aqueux, en présence d'un catalyseur acide, selon lequel l'acétalisation est réalisée par mélange des réactifs sous agitation, le mélange réactionnel est ensuite neutralisé par une base, la phase solide est séparée de la phase liquide et lavée à l'eau chaude, le rapport molaire initial du benzaldéhyde au sorbitol est inférieur à 2/1 et la température de réaction est inférieure à 45 ° C environ, lequel procédé est caractérisé par le fait que:

- le catalyseur acide est un acide phénylsulfonique ou naphtylsulfonique et
- le rapport molaire initial de l'acide phénylsulfonique ou de l'acide naphtylsulfonique au benzaldéhyde est supérieur à 0,6.

Selon un autre mode préféré de réalisation, l'invention a pour objet un procédé pour la préparation du bis(para-éthylbenzylidène)sorbitol [respectivement du bis(para-méthylbenzylidène)sorbitol] par acétalisation du sorbitol par le para-éthyl-benzaldéhyde (respectivement par le para-méthyl-benzaldéhyde) en milieu aqueux, en présence d'un catalyseur acide, selon lequel l'acétalisation est réalisée par mélange des réactifs sous agitation, le mélange réactionnel est ensuite neutralisé par une base, la phase solide est séparée de la phase liquide et lavée à l'eau chaude, le rapport molaire initial du para-éthyl-benzaldéhyde (respectivement du para-méthyl-benzaldéhyde) au sorbitol est inférieur à 2/1 et la température de réaction est inférieure à 45 ° C environ, lequel procédé est caractérisé par le fait que:

- le catalyseur acide est un acide phénylsulfonique ou naphtylsulfonique et
- le rapport molaire initial de l'acide phénylsulfonique ou de l'acide naphtylsulfonique au para-éthyl-benzaldéhyde (respectivement au para-méthyl-benzaldéhyde) est supérieur à 0,6.

Selon un autre mode préféré de réalisation, l'invention a pour objet un procédé pour la préparation du dibenzylidène-xylitol par acétalisation du xylitol par le benzaldéhyde en milieu aqueux, en présence d'un catalyseur acide, selon lequel l'acétalisation est réalisée par mélange des réactifs sous agitation, le mélange réactionnel est ensuite neutralisé par une base, la phase solide est séparée de la phase liquide et lavée à l'eau chaude, le rapport molaire initial du benzaldéhyde au xylitol est inférieur à 2/1 et la température de réaction est inférieure à 45 ° C environ, lequel procédé est caractérisé par le fait que:

- le catalyseur acide est un acide phénylsulfonique ou naphylsulfonique et
- le rapport molaire initial de l'acide phénylsulfonique ou naphtylsulfonique au benzaldéhyde est supérieur à 0,6.

Selon un mode préféré de réalisation de l'invention, l'alditol est mis en oeuvre sous la forme d'une solution aqueuse dont la concentration ne dépasse pas environ 30% et est de préférence comprise entre 20 et 30%. Au-dessus de 30%, la viscosité du milieu pourrait en effet devenir un facteur limitant de la réaction et, en dessous de 20%, la Société Demanderesse a observé que la durée de réaction était significativement augmentée.

Selon un autre mode préféré de réalisation de l'invention, la quantité d'aldéhyde benzoïque, notamment de benzaldéhyde, est telle que le rapport molaire initial aldéhyde benzoïque/alditol soit compris entre 1/1 et 1,95/1, de préférence entre 1,5/1 et 1,9/1 et, de préférence encore, entre 1,7/1 et 1,85/1.

De préférence, la quantité de catalyseur est choisie de telle sorte que le rapport molaire initial acide arylsulfonique/aldéhyde benzoïque soit compris entre 0,6/1 et 1,5/1, de préférence entre 0,6/1 et 1/1.

L'ordre d'introduction des réactifs et du catalyseur est indifférent selon la présente invention.

Le mélange aqueux tel qu'il vient d'être décrit dans sa composition initiale est porté, puis maintenu, sous agitation, à une température de réaction inférieure à 45 ° C environ, de préférence comprise entre 15 et 45 ° C, de préférence encore entre 20 et 40 ° C et plus particulièrement entre 30 et 40 ° C.

La durée de la réaction selon l'invention est généralement de l'ordre de 5 à 6 heures. Toutefois, pour des températures inférieures à environ 20 ° C, on a constaté qu'elle doit être augmentée et ce d'autant plus que la température diminue. En conséquence, dans un souci de rentabilité et de facilité de mise en oeuvre du procédé, on préfère travailler à une température au moins égale à 15 ° C environ. En tout état de cause, il va de soi que la température de réaction est choisie de telle sorte qu'on soit toujours initialement en

présence d'une phase liquide.

Le milieu réactionnel qui se présente sous forme d'une suspension aqueuse est ensuite neutralisé à l'aide d'un agent alcalin tel que l'hydroxyde de sodium, l'hydroxyde de potassium, le bicarbonate de sodium ou tout autre agent à activité alcaline, compatible avec le produit préparé.

On cherchera généralement à atteindre pour ladite suspension, un pH compris entre 7 et 7,5 environ, un léger excès d'agent alcalin n'ayant aucun effet susceptible de nuire à la bonne mise en oeuvre de la présente invention. Par tout moyen classique, on sépare ensuite entre elles les phases liquide et solide de la suspension aqueuse ainsi neutralisée. Ainsi, par filtration de ladite suspension aqueuse, on obtient un gâteau humide contenant le diacétal, par exemple du DBS, ledit gâteau étant ensuite soumis à un cycle au moins de lavage/filtration.

L'opération de lavage se fait de préférence à l'eau chaude (environ 60°C) et a pour but d'éliminer, entre autres, tout excès éventuel d'agent alcalin, tout sel résiduel formé à la suite de la neutralisation de la suspension aqueuse ainsi que toute impureté de type monoacétal, tel que le MBS, éventuellement présente ou tout alditol résiduel.

A partir de l'ultime étape de filtration, on récupère un gâteau humide, lequel est exempt de monoacétal (MBS par exemple) et ne contient que du diacétal (DBS par exemple) et d'éventuelles impuretés de type triacétal (TBS par exemple).

Le diacétal, notamment le DBS, le BEBS, le BMBS ou le DBX, ainsi préparé à partir d'un milieu réactionnel aqueux présente une pureté remarquable, dans tous les cas d'au moins 95 %.

Ce produit peut ensuite être séché afin d'en chasser l'eau résiduelle puis éventuellement broyé et tamisé, ces diverses opérations ne nécessitant aucunement la mise en oeuvre de moyens autres que ceux communément rencontrés dans l'art antérieur.

Comme on le voit, le procédé de l'invention ne nécessite la présence de solvant organique dans aucune de ses étapes opératoires et permet, de manière simple, économique et reproductible, de préparer une diacétal d'alditol, notamment du DBS, du BEBS, du BMBS ou du DBX, qui soit d'une pureté telle que, quelle que soit l'application à laquelle on destine ledit diacétal, il est possible de s'affranchir de toute étape complémentaire de purification de celui-ci, évitant ainsi la présence de traces de solvants organiques dans le produit fini.

Comme le montrent les exemples qui suivent, l'invention fournit outre un procédé permettant d'obtenir de façon générale du DBS, du BEBS, du BMBS ou du DBX ayant une pureté d'au moins 95 %, selon certains de ses modes de réalisation, un procédé de préparation d'un diacétal d'alditol en milieu aqueux selon lequel le diacétal obtenu est exempt de triacétal.

Eu égard à la technologie aqueuse connue dans l'art antérieur, le procédé selon l'invention permet notamment de s'affranchir de :

- toute étape au cours de laquelle le milieu réactionnel est porté à une température supérieure à 45°C environ,
- éventuellement toute étape de refroidissement,
- toute addition différée de catalyseur ou toute addition continue de réactif.

Les exemples suivants qui se rapportent à la préparation de diacétals selon l'invention et notamment du DBS sont destinés à illustrer et à mieux expliquer l'invention. Les exemples comparatifs désignés par "comp." dans les tableaux qui suivent sont destinés à mettre en évidence les avantages importants et inattendus apportés par le choix particulier des caractéristiques du procédé selon l'invention.

Dans les différents exemples du présent mémoire, on utilise les définitions suivantes :

Composition du gâteau avant lavage : rapport pondéral (exprimé en %) entre chacun des différents constituants du gâteau et la matière sèche dudit gâteau.

Pureté du diacétal : elle se définit comme suit

$$\text{pureté du diacétal} = \frac{\text{masse de diacétal}}{\text{masse de diacétal + triacétal}} \times 100.$$

Rendement massique en diacétal : il se définit comme suit

$$\text{rendement massique en diacétal} : = \frac{\text{masse de diacétal}}{\text{masse d'alditol mis en oeuvre}} \times 100$$

I. Influence du rapport molaire initial aldéhyde benzoïque/sorbitol sur la pureté du DBS préparé.

Conditions communes :

Dans le cadre de la présente étude, chacun des exemples est effectué conformément aux conditions opératoires suivantes :

| | |
|---|---|
| Concentration du sorbitol : | 25 % |
| Catalyseur : | acide paratoluènesulfonique (APTS) rapport molaire acide/sorbitol = 1,25 |
| Température : | 30 ° C |
| Durée : | 5 h 30. |

L'exemple décrit en détail ci-dessous concerne un mode de réalisation particulièrement avantageux de l'invention (exemple 4, tableau I).

Le mode opératoire utilisé dans cet exemple peut être pris en tant que protocole opératoire de référence.

Description détaillée de l'exemple 4 et protocole opératoire de référence :

Dans un réacteur cylindrique de 2 l muni d'une double enveloppe et d'un agitateur rotatif équipé d'une turbine à 3 pales, on introduit 728 g d'une solution aqueuse de sorbitol à 25 % de matière sèche (1 mole), 215 g d'acide paratoluènesulfonique (1,25 mole) et 190,8 g de benzaldéhyde (1,8 mole).

Ce mélange aqueux est porté, sous agitation, à la température de 30 ° C puis maintenu dans ces conditions pendant une durée de 5 heures et 30 minutes environ. Le milieu réactionnel ainsi obtenu est neutralisé par une solution d'hydroxyde de sodium à 10 % jusqu'à l'obtention d'un pH se situant au voisinage de 7,2, puis filtré sous vide sur un filtre de type BUCHNER.

Le gâteau de filtration résultant est alors remis en suspension dans de l'eau chaude (environ 60 ° C) puis filtré à nouveau. Le produit obtenu, qui présente une matière sèche d'environ 50 %, est ensuite séché à l'étuve sous vide pendant 8 heures à une température de 90 ° C, puis broyé.

On obtient ainsi 213,3 g d'une poudre de dibenzylidène-sorbitol (DBS) ne renfermant pas de tribenzylidène-sorbitol (TBS) (rendement massique en DBS : 117,2 %).

Dans cet exemple 4 le rapport molaire benzaldéhyde/sorbitol est égal à 1,8. Dans chacun des exemples 1 à 3 et 5, ainsi que dans les exemples comparatifs 1 et 2, on a fait varier ce rapport.

Les résultats obtenus sont rassemblés dans le tableau I qui suit.

**TABLEAU I**

| | | COMPOSITION DU GATEAU (%) AVANT LAVAGE | | | | | |
|---|---|---|---|---|---|---|---|
| Exemple n° | Benzaldéhyde/ sorbitol (rapport molaire) | Sorbitol | MBS | DBS | TBS | Pureté DBS % | Rendement DBS % |
| 1 | 1 | 13.5 | 3.1 | 83.4 | 0 | 100 | 70.1 |
| 2 | 1.5 | 8.8 | 3.1 | 88.1 | 0 | 100 | 103.6 |
| 3 | 1.7 | 7.6 | 3.1 | 89.3 | 0 | 100 | 107.1 |
| 4 | 1.8 | 5.4 | 2.5 | 92.1 | 0 | 100 | 117.2 |
| 5 | 1.9 | 4.4 | 2.6 | 89.0 | 4.0 | 95.7 | 128.0 |
| 1 (comp.) | 2.0 | 4.4 | 0 | 90.2 | 5.4 | 94.4 | 129.6 |
| 2 (comp.) | 3.0 | 6.5 | 0 | 71.3 | 22.2 | 76.3 | 91.8 |

Les résultats du tableau I montrent qu'avec un rapport molaire initial du benzaldéhyde au sorbitol égal ou supérieur au rapport stoechiométrique de 2, on n'obtient pas de DBS ayant une pureté satisfaisante.

Ces résultats montrent en outre que pour un rapport molaire initial benzaldéhyde/sorbitol non supérieur à 1,8 environ, le procédé selon l'invention permet de préparer du DBS exempt de TBS (pureté de 100 %).

II. Influence du rapport molaire initial catalyseur/benzaldéhyde sur la pureté du DBS préparé.

Les essais sont effectués conformément au protocole de référence sus-décrit en adoptant les conditions opératoires suivantes :

| | |
|---|---|
| Concentration du sorbitol : | 25 % |
| Rappot molaire : | benzaldéhyde/sorbitol = 1,8 |
| Catalyseur : | acide paratoluènesulfonique (APTS) |
| Température : | 30 ° C |
| Durée : | 5 h 30. |

Pour chacun des exemples comparatifs 3 et 4 et des exemples 6 à 8 selon l'invention, on fait varier le rapport catalytique acide paratoluènesulfonique/benzaldéhyde. Les valeurs obtenues sont rassemblées dans le tableau II ci-après.

TABLEAU II

| | | COMPOSITION DU GATEAU (%) AVANT LAVAGE | | | | | |
|---|---|---|---|---|---|---|---|
| Exemple n° | Acide/aldéhyde (rapport molaire) | Sorbitol | MBS | DBS | TBS | Pureté DBS % | Rendement DBS % |
| 3 (comp.) | 0,42 | 9,3 | 5,2 | 70,8 | 14,7 | 82,8 | 58,1 |
| 4 (comp.) | 0,55 | 10,8 | 4,2 | 76,9 | 8,1 | 90,5 | 80,0 |
| 6 | 0,64 | 5,4 | 3,7 | 90,9 | 0 | 100 | 107,7 |
| 7 | 0,69 | 5,4 | 2,5 | 92,1 | 0 | 100 | 117,2 |
| 8 | 0,77 | 4,3 | 1,8 | 93,9 | 0 | 100 | 118,0 |

Les résultats du tableau II montrent que pour obtenir une pureté en DBS satisfaisante, la proportion d'acide arylsulfonique utilisée doit être relativement importante. Plus précisément, le rapport molaire acide arylsulfonique/aldéhyde benzoïque initial doit être au moins égal à 0,6 environ.

III. Influence de la température réactionnelle sur la pureté du DBS préparé.

Les essais sont effectués conformément au protocole de référence sus-décrit en adoptant les conditions opératoires suivantes :

| | |
|---|---|
| Concentration du sorbitol : | 25 % |
| Rapport molaire benzaldéhyde/sorbitol = | 1,8 |
| Catalyseur : | acide paratoluènesulfonique (APTS) rapport molaire : acide/sorbitol = 1,25 |
| Durée : | 5 h 30. |

Dans les exemples 9 à 13 selon l'invention et l'exemple comparatif 5, on fait varier la température. Les valeurs obtenues sont rassemblées dans le tableau III ci-dessous.

## TABLEAU III

| | | COMPOSITION DU GATEAU (%) AVANT LAVAGE | | | | | |
|---|---|---|---|---|---|---|---|
| Exemple n° | Température réactionnelle °C | Sorbitol | MBS | DBS | TBS | Pureté DBS % | Rendement DBS % |
| 9 | 20 | 8,5 | 4,4 | 83,0 | 4,1 | 95,3 | 88,4 |
| 10 | 25 | 5,5 | 0 | 90,2 | 4,3 | 95,4 | 105,8 |
| 11 | 30 | 5,4 | 2,5 | 92,1 | 0 | 100 | 117,2 |
| 12 | 40 | 5,3 | 1,8 | 92,9 | 0 | 100 | 116,3 |
| 13 | 45 | 3,3 | 1,0 | 91,0 | 4,7 | 95,1 | 93,8 |
| 5 (comp.) | 50 | 1,9 | 0 | 68,3 | 29,8 | 69,6 | 36,1 |

Les résultats du tableau III montrent que lorsque la température réactionnelle est supérieure à 45 °C environ, la pureté du DBS formé est incompatible avec l'esprit de la présente invention en raison d'une trop forte proportion de TBS.

En revanche, une température réactionelle de l'ordre de 30 à 40 °C, dans les conditions indiquées plus haut, offre la possibilité remarquable de s'affranchir de toute formation de TBS et de préparer du DBS d'une extrême pureté.

Dans un essai réalisé à 15 °C, après 10 heures de réaction, les autres paramètres étant inchangés, on a obtenu les résultats suivants :

| | COMPOSITION DU GATEAU (%) AVANT LAVAGE | | | | | |
|---|---|---|---|---|---|---|
| Exemple n° | Sorbitol | MBS | DBS | TBS | Pureté DBS % | Rendement DBS % |
| 14 | 4,1 | 2,4 | 89 | 4,5 | 95,2 | 112,4 |

Ces résultats montrent que pour des températures inférieures à 20 °C, on obtient une pureté en DBS satisfaisante en laissant la réaction se poursuivre au-delà de 5 h30.

IV. Influence de la nature du catalyseur sur la pureté du DBS préparé.

Les essais sont effectués conformément au protocole de référence sus-décrit, en adoptant les conditions opératoires suivantes :

| | |
|---|---|
| Concentration du sorbitol : | 25 % |
| Rapport molaire benzaldéhyde/sorbitol = | 1,8 |
| Rapport molaire catalyseur/benzaldéhyde = | 0,69 |
| Température : | 30 °C |
| Durée : | 5 h 30. |

Dans les exemples 15 à 19 selon l'invention et les exemples comparatifs 6 à 12, on fait varier la nature du catalyseur, c'est-à-dire la nature de l'acide employé. Les résultats obtenus sont rassemblés dans le

tableau IV qui suit.

## TABLEAU IV

| Exemple n° | Nature de l'acide | Composition du gateau (%) avant lavage: Sorbitol | MBS | DBS | TBS | Pureté DBS % | Rendement DBS % |
|---|---|---|---|---|---|---|---|
| 15 | Paratoluènesulfonique | 5,4 | 2,5 | 92,1 | 0 | 100 | 117,2 |
| 16 | Benzènesulfonique | 5,3 | 0 | 94,7 | 0 | 100 | 111,6 |
| 17 | 5-sulfosalicylique | 9,2 | 0 | 90,8 | 0 | 100 | 101,0 |
| 18 | Naphtalènesulfonique | 0,7 | 0 | 99,3 | 0 | 100 | 109,0 |
| 19 | Dodécylbenzènesulfonique | 3,4 | 1,8 | 94,8 | 0 | 100 | 67,0 |
| 6 (comp.) | Méthanesulfonique | 5,6 | 0 | 45,2 | 49,2 | 47,9 | 19,0 |
| 7 (comp.) | Benzoïque | ABSENCE DE GATEAU RECUPERABLE | | | | | |
| 8 (comp.) | Paranitrobenzoïque | | | | | | |
| 9 (comp.) | Salicylique | | | | | | |
| 10 (comp.) | Succinique | | | | | | |
| 11 (comp.) | Orthophtalique | | | | | | |
| 12 (comp.) | Chlorhydrique | 3,3 | 0 | 52,4 | 44,3 | 54,2 | 24,6 |

Les résultats du tableau IV montrent l'influence décisive de la nature du catalyseur acide utilisé. Il est en particulier remarquable et surprenant de constater que l'acide benzènesulfonique donne d'excellents résultats (obtention de DBS exempt de TBS), alors que tant son homologue alcoylé, l'acide méthanesulfonique, que son homologue carboxylé, l'acide benzoïque donnent de mauvais résultats (mélange contenant une très forte proportion de TBS dans un cas, absence de gâteau récupérable dans l'autre cas).

Il convient de noter qu'une comparaison similaire peut être faite entre l'acide 5-sulfosalicylique et son homologue ne comportant pas de groupe acide sulfonique, l'acide salicylique.

L'acide naphtalènesulfonique (exemple 18) qui présente un groupement aryle "complexe", permet d'obtenir une pureté et un rendement pondéral en DBS comparables à ceux obtenus avec l'acide paratoluènesulfonique (APTS) (exemple 15).

En revanche, l'acide dodécylbenzènesulfonique (exemple 19) fournit une bonne pureté du DBS mais avec un rendement pondéral relativement faible. En conséquence, cet acide bien que pouvant être utilisé selon l'invention, n'est pas préféré.

La présence simultanée d'un groupement aryle tel que défini plus haut et d'un groupement acide sulfonique semble donc indispensable dans le cadre de l'invention.

V. Influence de la nature de l'alditol

Les essais sont effectués conformément au protocole de référence sus-décrit, en adoptant les conditions opératoires suivantes :

| | |
|---|---|
| Concentration de l'alditol : | 25 % |
| Rapport molaire benzaldéhyde/alditol : | 1,8 |
| Catalyseur : | acide paratoluènesulfonique |
| Rapport molaire acide/alditol : | 1,25 soit |
| Rapport molaire catalyseur/benzaldéhyde : | 0,69 |
| Température : | 30 °C |
| Durée de réaction : | 5 h 30. |

Dans les exemples 20 et 21 selon l'invention, on fait varier la nature de l'alditol.

L'exemple 20 correspond à l'exemple "de référence" selon l'invention (exemple 4 du tableau I, exemple 7 du tableau II, exemple 11 du tableau III et exemple 15 du tableau IV).

L'exemple 21 décrit la préparation du dibenzylidène-xylitol (DBX). L'invention permet d'obtenir ce composé avec un rendement pondéral très élevé de 172 % par rapport au poids de xylitol mis en oeuvre.

Certes le xylitol étant un alditol à 5 atomes de carbone (PM = 152), il ne peut donner lieu à la formation de triacétal, la pureté après lavage est donc "obligatoirement" de 100 %. Toutefois, il convient ici d'insister sur le fait que l'invention permet d'obtenir le DBX avec un rendement particulièrement élevé, ce qui montre que la réaction s'effectue avec une grande facilité et une sélectivité élevée [taux élevé de conversion du xylitol et peu de formation de monoacétal].

Les résultats obtenus sont rassemblés dans le tableau V qui suit.

## TABLEAU V

| | | COMPOSITION DU GATEAU (%) AVANT LAVAGE | | | | | |
|---|---|---|---|---|---|---|---|
| Exemple n° | Nature de l'alditol | Alditol | acétals mono | acétals di | acétals tri | Pureté diacétal % | Rendement diacétal % |
| 20 | sorbitol | 5,4 | 2,5 | 92,1 | 0 | 100 | 117,2 |
| 21 | xylitol | 0,7 | 0 | 99,3 | 0 | 100 | 172 |

VI. Influence de la nature de l'aldéhyde sur la préparation du diacétal (de sorbitol).

Les essais sont effectués conformément au protocole de référence sus-décrit, en adoptant les conditions opératoires suivantes :

| | |
|---|---|
| Concentration du sorbitol : | 25 % |
| Rapport molaire aldéhyde/sorbitol : | 1,8 |
| Catalyseur : | acide paratoluènesulfonique (APTS) |
| Rapport molaire acide/sorbitol : | 1,25 soit |
| Rapport molaire acide/benzaldéhyde : | 0.69 |
| Température : | 30 °C |
| Durée : | 5 h 30 |

Dans les exemples 22 à 25 selon l'invention, on fait varier la nature de l'aldéhyde, benzaldéhyde (exemple 22) et benzaldéhyde substitué sur le noyau phényle par au moins un substituant choisi parmi les groupes alcoyle inférieur avec 1 à 4 atomes de carbone, soit ici le groupe méthyle (exemple 23) et le groupe éthyle (exemple 24), l'un et l'autre en position para, ou par au moins un halogène, soit ici un atome de chlore en position para (exemple 25).

Les résultats obtenus sont rassemblés dans le tableau VI qui suit.

## TABLEAU VI

| | | COMPOSITION DU GATEAU (%) AVANT LAVAGE | | | | | |
|---|---|---|---|---|---|---|---|
| Exemple n° | Nature de l'aldéhyde | Sorbitol | acétals mono | di | tri | Pureté diacétal % | Rendement diacétal % |
| 22 | benzaldéhyde | 5,4 | 2,5 | 92,1 | 0 | 100 | 117,2 |
| 23 | P-Me benz. | 4,1 | 14,7 | 81,2 | 0 | 100 | 96,5 |
| 24 | P-Et benz. | 1,1 | 0 | 98,9 | 0 | 100 | 68,5 |
| 25 | P-Cl benz. | 0 | 82,1 | 17,9 | 0 | 100 | 32,0 |

Les exemples 23, 24 et 25 montrent que l'on peut obtenir un diacétal ayant une pureté d'au moins 95 % (en l'occurence 100 %) en mettant en oeuvre, en tant qu'aldéhyde benzoïque, un benzaldéhyde substitué (en position para) soit par un groupement méthyle (ex. 23) soit par un groupement éthyle (ex. 24) soit par un atome de chlore (ex. 25).

Le dérivé chloré fournit un produit qui, avant lavage, contient une forte proportion de monoacétal. Les dérivés halogénés qui peuvent être utilisés selon l'invention car ils conduisent à une très grande pureté (ici 100 %) ne sont donc pas préférés en raison du faible rendement obtenu, dû notamment à la formation d'une quantité importante de monoacétal.

VII. Influence du rapport molaire initial catalyseur/benzaldéhyde substitué sur la pureté et le rendement du diacétal préparé.

Les essais sont effectués conformément au protocole de référence sus-décrit, en adoptant les conditions opératoires suivantes :

| | |
|---|---|
| Concentration du sorbitol : | 25 % |
| Rapport molaire aldéhyde/sorbitol : | 1,8 |
| Catalyseur : | acide paratoluènesulfonique (APTS) |
| Température : | 30 ° C |
| Durée : | 5 h 30 |

Pour chacun des exemples 26 à 32 selon l' invention, on fait varier le rapport molaire acide paratoluènesulfonique/aldéhyde pour les deux aldéhydes suivants : p-méthyl-benzaldéhyde et p-éthyl-benzaldéhyde.

Les valeurs obtenues sont rassemblées dans les tableaux VII et VIII ci-après, respectivement.

TABLEAU VII

| Exemple n° | Acide/p-méthylbenzaldéhyde (rapport molaire) | COMPOSITION DU GATEAU (%) AVANT LAVAGE: Sorbitol | acétals mono | acétals di | acétals tri | Pureté diacétal % | Rendement diacétal % |
|---|---|---|---|---|---|---|---|
| 26 | 0,69 | 4,1 | 14,7 | 81,2 | 0 | 100 | 96,5 |
| 27 | 0,83 | 5,4 | 0 | 94,6 | 0 | 100 | 116,9 |
| 28 | 0,97 | 8,0 | 0 | 92,0 | 0 | 100 | 125,9 |

Note : le diacétal formé est le bis(para-méthylbenzylidène)sorbitol.

TABLEAU VIII

| Exemple n° | Acide/p-éthyl benzaldéhyde (rapport molaire) | COMPOSITION DU GATEAU (%) AVANT LAVAGE: Sorbitol | acétals mono | acétals di | acétals tri | Pureté diacétal % | Rendement diacétal % |
|---|---|---|---|---|---|---|---|
| 29 | 0,69 | 1,1 | 0 | 98,9 | 0 | 100 | 68,5 |
| 30 | 0,77 | 5,2 | 0 | 94,8 | 0 | 100 | 102,2 |
| 31 | 0,83 | 4,2 | 0 | 95,2 | 0 | 100 | 120,8 |
| 32 | 0,97 | 4,05 | 0 | 95,95 | 0 | 100 | 140,0 |

Note : le diacétal formé est le bis(paraéthylbenzylidène)sorbitol.

Les tableaux VII et VIII ci-dessus montrent qu'avec des dérivés du benzaldéhyde tels que ceux qui sont substitués par des groupements para-éthyle ou para-méthyle, on obtient un diacétal d'une pureté d'au moins 95 % (et en l'occurence de 100 %) avec des rapports molaires catalyseur (APTS)/aldéhyde benzoïque de 0,69 et plus.

De plus, avec des rapports molaires catalyseur/aldéhyde d'au moins 0,83, dans les deux cas on ne retrouve pas de monoacétal dans le gâteau obtenu avant lavage.

## Revendications

1. Diacétal résultant de la déshydrocondensation d'un alditol à 5 ou 6 atomes de carbone et d'un aldéhyde benzoïque, en particulier le benzaldéhyde éventuellement substitué par au moins un groupe alcoyle de 1 à 4 atomes de carbone ou par un halogène, à l'exclusion du bis(paraéthylbenzylidène)-sorbitol ou BEBS et du bis(para-méthylbenzylidène) sorbitol ou BMBS, ce diacétal qui présente une pureté d'au moins 95% et qui est exempt de traces de solvants organiques, étant susceptible d'être

préparé par mise en oeuvre du procédé selon lequel l'acétalisation est réalisée par mélange des réactifs sous agitation, le mélange réactionnel neutralisé ensuite par une base, la phase solide séparée de la phase liquide et lavée à l'eau chaude, le rapport molaire initial de l'aldéhyde benzoïque à l'alditol inférieur à 2/1 et la température de réaction inférieure à 45° C environ, ce procédé étant caractérisé par le fait que:

- le catalyseur acide est un acide arylsulfonique et
- le rapport molaire initial de l'acide arylsulfonique à l'aldéhyde benzoïque est supérieur à 0,6.

2. Diacétal selon la revendication 1, caractérisé par le fait qu'il s'agit du dibenzylidène-sorbitol ou DBS, du dibenzylidène-xylitol ou DBX, ou du bis(para-chlorobenzylidène)sorbitol.

3. Procédé pour la préparation d'un diacétal à partir d'un alditol comprenant 5 ou 6 atomes de carbone et d'un aldéhyde benzoïque en milieu aqueux, en présence d'un catalyseur acide, selon lequel l'acétalisation est réalisée par mélange des réactifs sous agitation, le mélange réactionnel neutralisé ensuite par une base, la phase solide séparée de la phase liquide et lavée à l'eau chaude, le rapport molaire initial de l'aldéhyde benzoïque à l'alditol inférieur à 2/1 et la température de réaction inférieure à 45° C environ, lequel procédé étant caractérisé par le fait que:

- le catalyseur acide est un acide arylsulfonique et
- le rapport molaire initial de l'acide arylsulfonique à l'aldéhyde benzoïque est supérieur à 0,6.

4. Procédé selon la revendication 3, caractérisé en ce que le catalyseur acide est un acide phénylsulfonique ou naphtylsulfonique.

5. Procédé selon la revendication 3 ou 4, caractérisé en ce que l'aldéhyde benzoïque est le benzaldéhyde

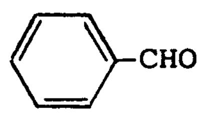

non substitué ou substitué sur le noyau phényle par au moins un substituant choisi parmi les groupes alcoyle inférieur avec 1 à 4 atomes de carbone, de préférence le groupe méthyle ou le groupe éthyle.

6. Procédé selon la revendication 3 ou 4 pour la préparation des diacétals de sorbitol, caractérisé en ce que l'aldéhyde benzoïque est un dérivé halogéné du benzaldéhyde

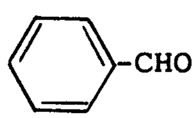 .

7. Procédé selon les revendications 3 et 4, caractérisé par le fait que le diacétal est le dibenzylidène-sorbitol, l'alditol est le sorbitol et l'aldéhyde benzoïque est le benzaldéhyde.

8. Procédé selon les revendications 3 à 5, caractérisé par le fait que le diacétal est le bis-(paraéthylbenzylidène)sorbitol, l'alditol est le sorbitol et l'aldéhyde benzoïque est le para-éthyl-benzaldéhyde.

9. Procédé selon les revendications 3 à 5, caractérisé par le fait que le diacétal est le bis-(paraméthylbenzylidène)sorbitol, l'alditol est le sorbitol et l'aldéhyde benzoïque est le para-méthyl-benzaldéhyde.

10. Procédé selon les revendications 3 à 5, caractérisé par le fait que le diacétal est le dibenzylidène-xylitol, l'alditol est le xylitol et l'aldéhyde benzoïque est le benzaldéhyde.

**11.** Procédé selon les revendications 3, 4 et 6, caractérisé par le fait que le diacétal est le bis-(parachlorobenzylidène)sorbitol, l'alditol est le sorbitol et l'aldéhyde benzoïque est le para-chloro-benzaldéhyde.

**12.** Procédé selon l'une des revendications 4 à 11, caractérisé par le fait que l'acide de type phénylsulfonique ou naphtylsulfonique utilisé est choisi parmi l'acide paratoluènesulfonique, l'acide benzènesulfonique, l'acide 5-sulfosalicylique et l'acide naphtalènesulfonique.

**13.** Procédé selon l'une des revendications 3 à 12, caractérisé par le fait que l'alditol est mis en oeuvre sous la forme d'une solution aqueuse dont la concentration ne dépasse pas 30% et est de préférence comprise entre 20 et 30%.

**14.** Procédé selon l'une des revendications 3 à 13, caractérisé par le fait que le rapport molaire initial de l'aldéhyde benzoïque à l'alditol est compris entre 1/1 et 1,95/1, de préférence entre 1,5/1 et 1,9/1, de préférence encore entre 1,7/1 et 1,85/1.

**15.** Procédé selon l'une des revendications 3 à 14, caractérisé par le fait que le rapport molaire initial acide arylsulfonique/aldéhyde benzoïque est compris entre 0,6/1 et 1,5/1, de préférence entre 0,6/1 et 1/1.

**16.** Procédé selon l'une des revendications 3 à 15, caractérisé par le fait que la température de réaction est comprise entre 15 et 45 ° C, de préférence entre 20 et 40 ° C, de préférence encore entre 30 et 40 ° C.

**17.** Procédé selon l'une des revendications 3 à 16, caractérisé en ce que le milieu réactionnel est neutralisé à la fin de la réaction au moyen d'un agent alcalin, choisi de préférence parmi l'hydroxyde de sodium, l'hydroxyde de potassium et le bicarbonate de sodium jusqu'à un pH compris entre 7 et 7,5 environ.

**Claims**

**1.** Diacetal resulting from the dehydrocondensation of an alditol having 5 or 6 carbon atoms and of a benzoic aldehyde, particularly benzaldehyde possibly substituted by at least one alkyl group having 1 to 4 carbon atoms or by a halogen, except bis(para-éthylbenzylidene)sorbitol or BEBS and bis (para-methylbenzylidene)sorbitol or BMBS, said diacetal which has a purity of at least 95% and which is free of traces of organic solvents, being obtainable by way of the process according to which the acetalization is carried out by mixing the reactants under stirring, the reaction mixture being then neutralized by a base, the solid phase being separated from the liquid phase and washed with warm water, the initial molar ratio of the benzoic aldehyde to the alditol being lower than 2/1 and the reaction temperature bein lower than about 45 ° C, said process being characterized by the fact that:
- the acid catalyst is an arylsulfonic acid and
- the initial molar ratio of the arylsulfonic acid to the benzoic aldehyde is higher than 0.6.

**2.** Diacetal according to claim 1, characterized by the fact that it is dibenzylidenesorbitol or DBS, dibenzylidenexylitol or DBX, or bis(para-chlorobenzylidene)sorbitol.

**3.** Process for preparing a diacetal starting from an alditol comprising 5 or 6 carbon atoms and from a benzoic aldehyde in aqueous medium, in the presence of an acid catalyst, according to which the acetalization is carried out by mixing the reactants under stirring, the reaction mixture is then neutralized by a base, the solid phase is separated from the liquid phase and washed with warm water, the initial molar ratio of the benzoic aldehyde to the alditol is lower than 2/1 and the reaction temperature is lower than about 45 ° C, said process being characterized by the fact that:
- the acid catalyst is an arylsulfonic acid and
- the initial molar ratio of the arylsulfonic acid to the benzoic aldehyde is higher than 0.6.

**4.** Process according to claim 3, characterized by the fact that the acid catalyst is a phenylsulfonic or naphthylsulfonic acid.

**5.** Process according to claim 3 or 4, characterized by the fact that the benzoic aldehyde is benzaldehyde

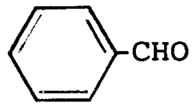

unsubstituted or substituted on the phenyl nucleus by at least one substituent selected from the lower alkyl groups having 1 to 4 carbon atoms, preferably the methyl or the ethyl group.

**6.** Process according to claim 3 or 4 for preparing sorbitol diacetals, characterized by the fact that the benzoic aldehyde is a halogenated derivative of benzaldehyde

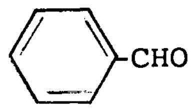

**7.** Process according to claims 3 and 4, characterized by the fact that the diacetal is dibenzylidenesorbitol, the alditol is sorbitol and the benzoic aldehyde is benzaldehyde.

**8.** Process according to claims 3 to 5, characterized by the fact that the diacetal is bis(para-ethylbenzylidene)sorbitol, the alditol is sorbitol and the benzoic aldehyde is para-ethylbenzaldehyde.

**9.** Process according to claims 3 to 5, characterized by the fact that the diacetal is bis-(paramethylbenzylidene)sorbitol, the alditol is sorbitol and the benzoic aldehyde is para-methylbenzaldehyde.

**10.** Process according to claims 3 to 5, characterized by the fact that the diacetal is dibenzylidenexylitol, the alditol is xylitol and the benzoic aldehyde is benzaldehyde.

**11.** Process according to claims 3, 4 and 6, characterized by the fact that the diacetal is bis-(parachlorobenzylidene)sorbitol, the alditol is sorbitol and the benzoic aldehyde is para-chloro-benzaldehyde.

**12.** Process according to one of claims 4 to 11, characterized by the fact that the acid of the phenylsulfonic or naphthylsulfonic type used is selected from among paratoluenesulfonic acid, benzenesulfonic acid, 5-sulfosalicylic acid and naphthalenesulfonic acid.

**13.** Process according to one of claims 3 to 12, characterized by the fact that the alditol is used in the form of an aqueous solution, the concentration of which does not exceed 30% and is preferably comprised between 20 and 30%.

**14.** Process according to one of claims 3 to 13, characterized by the fact that the initial molar ratio of the benzoic aldehyde to the alditol is comprised between 1/1 and 1.95/1, preferably between 1.5/1 and 1.9/1 and still preferably between 1.7/1 and 1.85/1.

**15.** Process according to one of claims 3 to 14, characterized by the fact that the initial molar ratio arylsulfonic acid/benzoic aldehyde is comprised between 0.6/1 and 1.5/1, preferably between 0.6/1 and 1/1.

**16.** Process according to one of claims 3 to 15, characterized by the fact that the reaction temperature is comprised between 15 and 45 ° C, preferably between 20 and 40 ° C and still preferably between 30 and 40 ° C.

**17.** Process according to one of claims 3 to 16, characterized by the fact that, at the end of the reaction, the reaction medium is neutralized by means of an alkaline agent preferably selected from among

sodium hydroxide, potassium hydroxide and sodium bicarbonate until a pH value comprised between about 7 and 7.5.

**Patentansprüche**

1. Diacetal, das das Ergebnis einer Dehydrokondensation eines Aldits mit 5 oder 6 Kohlenstoffatomen und eines Benzaldehyds, insbesondere von Benzaldehyd, ist, der gegebenenfalls durch wenigstens eine Alkylgruppe mit 1 bis 4 Kohlenstoffatomen oder durch ein Halogenatom substituiert ist, unter Ausschluß des Bis(para-ethylbenzyliden)sorbits oder BEBS und des Bis(para-methylbenzyliden)sorbits oder BMBS, wobei dieses Diacetal einen Reinheitsgrad von wenigstens 95 % aufweist und auch von Spuren organischer Lösungsmittel frei ist, und welches Diacetal durch Anwendung eines Verfahrens hergestellt werden kann, gemäß welchem Verfahren die Acetalisierung durch Vermischen der Reaktionskomponenten unter Rühren vorgenommen wird, wobei das Reaktionsgemisch anschließend durch eine Base neutralisiert wird, die feste Phase von der flüssigen Phase abgetrennt und mit warmem Wasser gewaschen wird, und wobei das Anfangsmolverhältnis zwischen dem Benzaldehyd und dem Aldit unter 2/1 liegt, und wobei die Reaktionstemperatur unter etwa 45° C liegt, welches Verfahren dadurch gekennzeichnet ist,
   - daß der saure Katalysator eine Arylsulfonsäur ist, und
   - daß das aufängliche Molverhältnis zwischen der Arylsulfonsäure und dem Benzaldehyd größer als 0,6 ist.

2. Diacetal nach Anspruch 1, dadurch gekennzeichnet, daß es sich um Dibenzylidensorbit oder DBS, um Dibenzylidenxylit oder DBX oder um Bis(para-chlorbenzyliden)sorbit handelt.

3. Verfahren zur Herstellung eines Diacetals, ausgehend von einem Aldit mit 5 oder 6 Kohlenstoffatomen und einem Benzaldehyd in wässerigem Medium, in Gegenwart eines sauren Katalysators, gemäß welchem Verfahren die Acetalisierung durch Vermischen der Reaktionskomponenten unter Rühren vorgenommen wird, wobei das Reaktionsgemisch anschließend durch eine Base neutralisiert wird, die feste Phase von der flüssigen Phase abgetrennt und mit warmem Wasser gewaschen wird, und wobei das Anfangsmolverhältnis zwischen dem Benzaldehyd und dem Aldit unter 2/1 liegt, und wobei die Reaktionstemperatur unter etwa 45° C liegt, welches Verfahren dadurch gekennzeichnet ist,
   - daß der saure Katalysator eine Arylsulfonsäure ist, und
   - daß das anfängliche Molverhältnis zwischen der Arylsulfonsäure und dem Benzaldehyd größer als 0,6 ist.

4. Verfahren nach Anspruch 3, dadurch gekennzeichnet, daß der saure Katalysator eine Phenyl- oder Naphthylsulfonsäure ist.

5. Verfahren nach Anspruch 3 oder 4, dadurch gekennzeichnet, daß der Benzaldehyd der Benzaldehyd

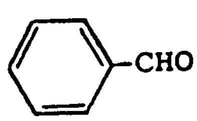

ist, welcher unsubstituiert ist oder welcher am Phenylkern durch wenigstens einen Substituenten substituiert ist, der aus niedrigen Alkylgruppen mit 1 bis 4 Kohlenstoffatomen, vorzugsweise aus einer Methyl- oder Ethylgruppe, ausgewählt ist.

6. Verfahren nach Anspruch 3 oder 4 zur Herstellung von Diacetalen des Sorbits, dadurch gekennzeichnet, daß der Benzaldehyd ein halogeniertes Derivat des Benzaldehydes

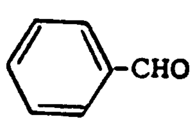

ist.

**7.** Verfahren nach den Ansprüchen 3 und 4, dadurch gekennzeichnet, daß das Diacetal Dibenzylidensorbit ist, der Aldit Sorbit ist, und der Benzaldehyd Benzaldehyd ist.

**8.** Verfahren nach den Ansprüchen 3 bis 5, dadurch gekennzeichnet, daß das Diacetal Bis(para-ethylbenzyliden)sorbit ist, der Aldit Sorbit ist und der Benzaldehyd para-Ethylbenzaldehyd ist.

**9.** Verfahren nach den Ansprüchen 3 bis 5, dadurch gekennzeichnet, daß das Diacetal Bis(para-methylbenzyliden)sorbit, der Aldit Sorbit ist und der Benzaldehyd para-Methylbenzaldehyd ist.

**10.** Verfahren nach den Ansprüchen 3 bis 5, dadurch gekennzeichnet, daß das Diacetal Dibenzylidenxylit ist, der Aldit Xylit ist, und der Benzaldehyd Benzaldehyd ist.

**11.** Verfahren nach den Ansprüchen 3, 4 und 6, dadurch gekennzeichnet, daß das Diacetal Bis(parachlorbenzyliden)sorbit ist, der Aldit Sorbit ist und der Benzaldehyd parr-Chlorbenzaldehyd ist.

**12.** Verfahren nach einem der Ansprüche 4 bis 11, dadurch gekennzeichnet, daß die verwendete Säure vom Typus der Phenyl- oder Naphthylsulfonsäure aus para-Toluolsulfonsäure, Benzolsulfonsäure, 5-Sulfosalicylsäure und Naphthalinsulfonsäure ausgewählt ist.

**13.** Verfahren nach einem der Ansprüche 3 bis 12, dadurch gekennzeichnet, daß der Aldit in der Form einer wässerigen Lösung eingesetzt wird, deren Konzentration nicht höher als 30 % ist und vorzugsweise zwischen 20 und 30 % liegt.

**14.** Verfahren nach einer der Ansprüche 3 bis 13, dadurch gekennzeichnet, daß das anfängliche Molverhältnis zwischen dem Benzaldehyd und dem Aldit zwischen 1/1 und 1,95/1, vorzugsweise zwischen 1,5/1 und 1,9/1 und in höherem Maße bevorzugt zwischen 1,7/1 und 1,85/1 liegt.

**15.** Verfahren nach einem der Ansprüche 3 bis 14, dadurch gekennzeichnet, daß das anfängliche Molverhältnis zwischen der Arylsulfonsäure und dem Benzaldehyd zwischen 0,6/1 und 1,5/1, vorzugsweise zwischen 0,6/1 und 1/1, liegt.

**16.** Verfahren nach einem der Ansprüche 3 bis 15, dadurch gekennzeichnet, daß die Reaktionstemperatur zwischen 15 und 45° C, vorzugsweise zwischen 20 und 40° C, und in höherem Maße bevorzugt zwischen 30 und 40° C liegt.

**17.** Verfahren nach einem der Ansprüche 3 bis 16, dadurch gekennzeichnet, daß das Reaktionsmedium am Ende der Reaktion mit Hilfe eines alkalischen Mittels neutralisiert wird, welches vorzugsweise aus Natriumhydroxid, Kaliumhydroxid und Natriumbicarbonat ausgewsählt ist, und zwar so lange, bis ein pH-Wert zwischen 7 und etwa 7,5 erreicht worden ist.